(19) Europäisches Patentamt
European Patent Office
Office européen des brevets

(11) Publication number: **0 206 138**
**B1**

# (12) EUROPEAN PATENT SPECIFICATION

(45) Date of publication of patent specification: 08.03.89

(21) Application number: 86108015.8

(22) Date of filing: 12.06.86

(51) Int. Cl.$^4$: **C 07 H 15/252,** A 61 K 31/70, C 12 P 19/56 // (C12P19/56, C12R1:03)

## (54) Anthracycline compounds, a process for their preparation and their use as medicaments.

(30) Priority: 24.06.85 JP 137193/85

(43) Date of publication of application: 30.12.86 Bulletin 86/52

(45) Publication of the grant of the patent: 08.03.89 Bulletin 89/10

(84) Designated Contracting States: AT BE CH DE FR GB IT LI LU NL SE

(56) References cited:
GB-A-2 060 609

JOURNAL OF ANTIBIOTICS, vol. 36, no. 8, August 1983, pages 83-108, Tokyo, JP; & JP - A - 81 90 098 (DAIICHI SEIYAKU) 21-07-1981

JOURNAL OF ANTIBIOTICS, vol. 30, no. 7, July 1977, pages 622-624; Y. TAKAHASHI: "The structure of baumycins A1,A2,B1,B2,C1,C2"

(73) Proprietor: MICROBIAL CHEMISTRY RESEARCH FOUNDATION
14-23, Kamiosaki 3 Chome Shinagawa-ku
Tokyo 141 (JP)

(72) Inventor: Umezawa, Hamao, Prof.
23, Toyotama-kita 4-chome
Nerima-ku Tokyo (JP)
Inventor: Takeuchi, Tomio
701A New Fuji Mansion 1-11 Higashi-gotanda 5-chome
Shinagawa-ku Tokyo (JP)
Inventor: Sawa, Tsutomu
6-7, Ryosei 4-chome
Ayase-city Kanagawa Prefecture (JP)
Inventor: Naganawa, Hiroshi
17, Denenchofu-honcho 3-chome Ota-ku
Tokyo (JP)
Inventor: Hamada, Masa
No. 405, Shuwa Residence 26, Naito-cho 1-chome
Shinjuku-ku Tokyo (JP)
Inventor: Takahashi, Yoshikazu
3-2-3, Sakuragaoka 3-chome
Tama-city Tokyo (JP)
Inventor: Imoto, Masaya
18-14-101, Tokiwa 7-chome
Urawa-city Saitama Prefecture (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 206 138 B1

(72) Inventor: **Odagawa, Atsuo**
**1365-3 Soja, Sojamachi**
**Maebashi-city Gunma Prefecture (JP)**
Inventor: **Uchida, Takeshi**
**3703-5-A-202, Ishiharamachi**
**Takasaki-city Gunma Prefecture (JP)**

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80 (DE)**

**Description**

The present invention relates to novel anthracycline compounds.

Anthracycline compounds such as adriamycin, daunomycin and acranomycin are important cancer-control antibiotics which are extensively used in cancer chemotherapy. Concerted efforts are being made by many researchers to develop various analogs and derivatives of these anthracycline compounds.

The physiological activities of most compounds are largely dependent on their chemical structures. Anthracycline compounds are not an exception and there exists an ever increasing demand for developing derivatives that differ from the existing compounds with respect to the type of the aglycon and sugar portions, and to the substituents on such portions.

The present invention relates therefore to anthracycline compounds (barminomycins), having the following formula (I):

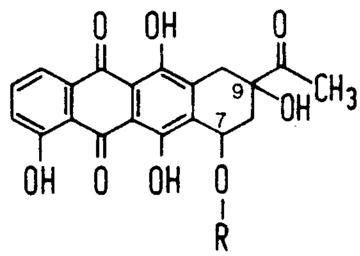

(I)

where R is any one of the following groups (a) to (d):

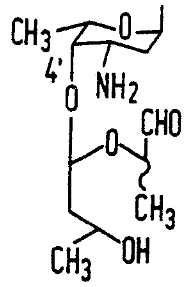

(a)

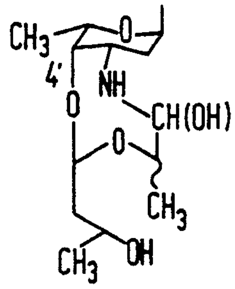

(b)

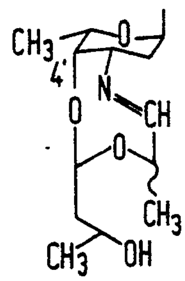

(c)

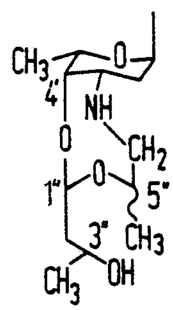

(d)

or acid addition salts thereof.

The anthracycline compounds of the present invention have the chemical structure represented by the formula (I). The substituent R can be (a), (b) (c) or (d), and each of them exists in two stereoisomeric forms with respect to the asymmetric carbon at 5″ position. A compound having (a), (b) or (c) as the substituent R is readily interconvertible in a stereospecific manner and usually exists in the form of a mixture of two of such groups in equilibrium, as shown below:

(a) (b) (c)

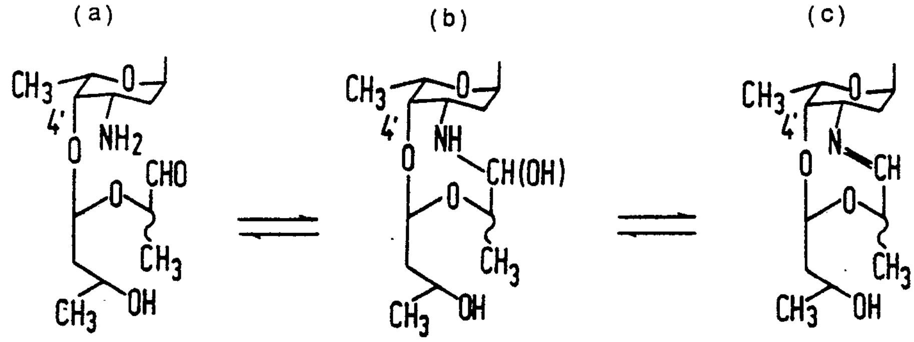

(aldehyde + primary amine type) (carbinolamine type) (imine type)

The equilibrium shown above has a tendency to shift toward (c) in organic solvents and toward (a) in the presence of water and acids.

Since two stereoisomers exist for the substituent R, the tautomer can also exist in one of the two forms, which will hereunder be referred to as barminomycin I and barminomycin II, respectively. The compound of the present invention having (d) as substituent R may be obtained by reducing barminomycin I or II (as will be described later in this specification); the compound obtained by reducing barminomycin I with $NaBH_3CN$ and the one obtained by reducing barminomycin II with $NaBH_3CN$ are hereunder referred to as barminomycin Ir and barminomycin IIr, respectively.

Both barminomycin I and II are currently obtained only by means of cultivating microorganisms and their chemical structures have been determined as follows by the sequence of analysis shown in Fig. 1.

Both barminomycin I and II present a main ion peak at m/z = 639 in FD—MS, and the two compounds are in close agreement in terms of absorption in the ultraviolet and visible ranges, absorption in the infrared range and the $^1H$—NMR spectrum. However, in view of the differences that exist with respect to the Rf values on TLC and specific rotations, it has been suggested that the compounds are two different stereoisomers. Both compounds yielded a red aglycon by heating at 85°C for 30 minutes in 0.1 N HCl, and as a result of $^1H$—NMR spectrum, mass spectrum and other analyses, this aglycon was identified as carminomycinone (Formula *2*) (see Journal of Antibiotics, *29*, 469—471, 1976). The aglycon from each compound also yielded a red glycoside by partial hydrolysis at 30°C in 1% sulfuric acid. Each of the resulting glycosides was in close agreement with an authentic sample of carminomycin I (Formula *3*) in terms of the Rf values on silica gel TLC, specific rotation and $^1H$—NMR spectra (see Journal of Antibiotics, *27*, 254—259, 1974).

When barminomycin I was dissolved in a mixture of methanol and 1 N aqueous acetic acid (2:1) and the solution reduced with $NaBH_3CN$, two red glycosides formed. On the basis of data on specific rotation ($[\alpha]_D^{27}$ + 178° (c 0.02, $CHCl_3$); documented as +170.4° (c 0.053, $CHCl_3$)), FD—MS (m/z = 660 M + $H)^+$) and $^1H$—NMR spectrum (see Table 1), one of the two glycosides was identified as carminomycin III (Formula *4*) (identical with 4-hydroxy baumycin $A_1$ or rubeomycin $A_1$; see Antibiotiki, 489—492, 1980; Journal of Antibiotics, *34*, 774—776, 1981; and Journal of Antibiotics, *34*, 938—950, 1981). The other reduced form (barminomycin Ir) was fairly close to the first glycoside except that it gave a main ion peak at m/z = 642 $(M + H)^+$ in FD—MS, that 33 carbon atoms were identified in $^{13}C$—NMR spectrum (Fig. 8) and that chemical shifts of carbon at 3′ position (51.6 ppm) and 6″ position (52.8 ppm) in the $^{13}C$—NMR spectrum changed appreciably as compared with the shifts for carminomycin III. Analysis of the $^1H$—NMR spectrum of barminomycin Ir (Fig. 5) revealed that it had the structure represented by Formula *5*.

When barminomycin II was likewise reduced with $NaBH_3CN$ carminomycin II (Formula *4*) (identical with 4-hydroxy baumycin $A_2$ or rubeomycin A; see the references listed for carminomycin III) ($[\alpha]_D^{27}$ + 124° (c 0.015, $CHCl_3$); documented as 120.6° (c 0.199, $CHCl_3$); m/z = 660 $(M + H)^+$ in FD—MS; and $^1H$—NMR spectrum (Table 1)) and barminomycin IIr were obtained. The latter compound was found to have the structure represented by formula *5* in view of its FD—MS (m/z = 642 $(M + H)^+$) and $^1H$—NMR spectra data (Fig. 7). It should, however, be mentioned that barminomycin Ir and IIr would be two stereoisomers at 5″ position in consideration of the differences that exist typically with respect to Rf values on silica gel TLC and specific rotation.

These facts and the physicochemical properties of the two compounds (to be described hereinafter) indicate that each of barminomycin I and barminomycin II is one of the compounds represented by Formula *1* or a tautomer thereof and that the two compounds are stereoisomers at 5″ position. These structures of barminomycin I and II are also supported by the fact that they are interconvertible when they are stored in the form of solutions, etc.

TABLE 1
$^{1}H$—NMR Spectral Data for Carminomycin III and II
(400 MHz in d—$CHCl_3$)

| Assignment | Carminomycin III | | Carminomycin II | |
|---|---|---|---|---|
| | δ (ppm) | signal pattern | δ (ppm) | signal pattern |
| 7″—$CH_3$ | 1.06 | d | 1.17 | d |
| 4″—$CH_3$ | 1.23 | d | 1.20 | d |
| 6′—$CH_3$ | 1.32 | d | 1.30 | d |
| 2′—$CH_3$ | ~1.80 | m | ~1.90 | m |
| 2″—$CH_2$ | 1.85 | m | 1.86 | m |
| 8—Hax | 2.08 | dd | 2.09 | dd |
| 8—Heq | 2.29 | ddd | 2.31 | ddd |
| $COCH_3$ | 2.42 | s | 2.41 | s |
| 10—Hax | 2.98 | d | 2.94 | d |
| 3′—H | 3.09 | m | 3.29 | m |
| 10—Heq | 3.23 | dd | 3.20 | dd |
| 6″—$H_B$ | 3.41 | br d | } 3.54 | m |
| 6″—$H_A$ | 3.51 | dd | | |
| 5″—H | 3.80 | m | ~3.84 | m |
| 4′—H | 3.93 | br s | 3.80 | m |
| 5′—H | 4.14 | dq | 4.14 | dq |
| 3′—H | 4.17 | m | 4.23 | m |
| 1′—H | 4.74 | t | 4.85 | dd |
| 7—H | 5.20 | br s | 5.18 | br s |
| 1′—H | 5.44 | dd | 5.48 | dd |
| 3—H | 7.31 | dd | 7.26 | dd |
| 2—H | 7.71 | dd | 7.67 | dd |
| 1—H | 7.87 | dd | 7.77 | dd |

Physicochemical Properties of Barminomycin

Barminomycin I and II in accordance with the present invention and their respective reduced forms, barminomycin Ir and IIr, have the physicochemical data shown in Table 2. The samples of barminomycin I and II used in the measurements were the tautomers prepared in Example 2 to be described hereinafter.

TABLE 2
Physicochemical Data of Barminomycin Compounds

| | I | II | Ir | IIr |
|---|---|---|---|---|
| 1. Color | red orange | red orange | red orange | red orange |
| 2. FD-MS, m/z | 639 $(M)^+$ | 639 $(M)^+$ | 642 $(M + H)^+$ | 642 $(M + H)^+$ |
| 3. Melting point | — | — | 129 ~ 134°C | 124 ~ 132°C |
| 4. Specific rotation | +235° (c 0.017, $CHCl_3$) | +184° (c 0.025, $CHCl_3$) | +294° (c 0.043, $CHCl_3$) | +187° (c 0.023, $CHCl_3$) |
| 5. Absorption spectrum for UV and visible bands λmax, nm ($E^{1\%}_{1\,cm}$) in 0.1 N HCl — 90% MeOH | 233 (712)<br>253 (505)<br>291 (150)<br>491 (275)<br>525 (180) | 233 (712)<br>253 (499)<br>291 (145)<br>491 (270)<br>525 (176) | 233 (666)<br>253 (489)<br>290 (145)<br>491 (272)<br>525 (179) | 233 (633)<br>253 (486)<br>290 (148)<br>490 (270)<br>525 (177) |
| 6. IR absorption spectrum | Fig. 2 (in d-$CHCl_3$) | Fig. 3 (in d-$CHCl_3$) | Fig. 4 (as KBr tablet) | Fig. 5 (as KBr tablet) |
| 7. $^1$H-NMR spectrum | Table 3 | Table 3 | Fig. 5 | Fig. 7 |
| 8. Rf values on silica gel TLC*[1] | 0.42 | 0.35 | 0.34 | 0.29 |
| 9. $^tR$*[2] in HPLC | 7.0 min | 8.5 min | 7.6 min | 7.2 min |

| | I | II | Ir | IIr |
|---|---|---|---|---|
| 10. Stability | Decomposed on silica gel and during concentration procedures<br><br>Instable to acids and light | Decomposed on silica gel and during concentration procedures<br><br>Instable to acids and light | Instable to acids and light | Instable to acids and light |
| 11. Solubility | | | | |
| (1) Soluble | chloroform, ethyl acetate, ethanol, methanol, acetonitrile, dimethyl sulfoxide | chloroform, ethyl acetate, ethanol, methanol, acetonitrile, dimethyl sulfoxide | chloroform, ethyl acetate, ethanol, methanol, acetonitrile, dimethyl sulfoxide | chloroform, ethyl acetate, ethanol, methanol, acetonitrile, dimethyl sulfoxide |
| (2) Sparingly soluble | water, hexane, petroleum ether | water, hexane, petroleum ether | water, hexane, petroleum ether | water, hexane, petroleum ether |

(Notes) *1 Developing solvent: chloroform-methanol-acetic acid-water (60:10:1:1)
*2 Column: Nucleosil $_5C_{18}$ (4.6 × 250 mm) (product of M-Nagel)
Eluant: acetonitrile-0.2% aqueous phosphoric acid (40:60)
Flow rate: 1.5 ml/min

TABLE 3
$^{1}$H-NMR Spectral Data for Barminomycin I and II
(400 MHz, in 2:1 d-$CHCl_3$/d-MeOH)

| Barminomycin I | | | Barminomycin II | | |
|---|---|---|---|---|---|
| δ (ppm) | Integration | Signal pattern | δ (ppm) | Integration | Signal pattern |
| 1.21 | 3H | d | | | |
| 1.23 | 3H | d | 1.18 ~ 1.33 | 9H | dx3 |
| 1.27 | 3H | d | | | |
| 1.72 | 2H | m | 1.75 ~ 1.98 | 4H | m |
| 1.89 | 2H | m | | | |
| 2.15 | 1H | dd | 2.13 | 1H | dd |
| 2.37 | 1H | ddd | 2.38 | 1H | ddd |
| 2.43 | 3H | s | 2.43 | 3H | s |
| 3.07 | 1H | d | 3.05 | 1H | d |
| 3.18 | 1H | m | ~3.15 | 1H | m |
| 3.23 | 1H | dd | 3.21 | 1H | dd |
| ~3.53 | 1H | m | 3.47 | 1H | m |
| 3.60 | 1H | br s | 3.57 | 1H | br s |
| 4.03 | 1H | m | 3.96 | 1H | m |
| 4.17 | 1H | dq | 4.20 | 1H | dq |
| 5.24 | 1H | dd | 5.23 | 1H | dd |
| 5.47 | 1H | dd | 5.47 | 1H | dd |
| 7.35 | 1H | dd | 7.33 | 1H | dd |
| 7.76 | 1H | dd | 7.75 | 1H | dd |
| 7.91 | 1H | dd | 7.90 | 1H | dd |

Preparation of Barminomycin

As already mentioned, the anthracycline compounds, barminomycin I and II, are currently produced only by means of cultivating microorganisms. They may, however, be produced by chemical synthetic or microbiological modification of analogous compounds, or by fully chemical synthetic procedures.

In producing the end compound of the present invention by cultivation of a microorganism, one that belongs to the genus *Actinomadura* and which has the ability to produce the anthracycline compound, barminomycin I or II. The present inventors have established that the strain MG463-yF4 which they have isolated has the ability to produce barminomycin I and II, but it should be noted that other suitable strains may be isolated from nature by conventional techniques for isolating antibiotic-producing microorganisms. It is also possible to enhance the barminomycin I- or II-producing ability of the strain MG463-yF4 and other barminomycin I- or II-producing microorganisms by treating them with radiation or by subjecting them to other suitable treatments. Microorganisms having the ability to produce barminomycin I or II may be induced by genetic engineering techniques wherein the gene DNA harboring genetic information that is related to barminomycin production is introduced by transformation, cell fusion or other suitable methods.

Barminomycin Ir and IIr are prepared by reducing barminomycin I and II, respectively, using chemical synthetic procedures.

The strain MG463-yF4 that has been identified by the present inventors as a microorganism having the ability to produce the anthracycline compound, barminomycin, is specifically described below.

(A) Origin and Accession Number

The strain MG463-yF4 is an actinomycete isolated by the Institute of Microbial Chemistry in October 1982 from soil sampled in Nangoku-shi, Kochi, Japan. This strain was deposited with the Fermentation Research Institute, the Agency of Industrial Science and Technology on November 24, 1983 and was given the accession number, FERM-P No. 7352. (= FERM-BP 1044).

(B) Mycological Properties

(1) Morphology

Strain MG463-yF4 as observed under a microscope has a fairly long mycelium extending from a branched aerial hypha.

The aerial mycelium bears a hooked chain of spores and a pseudo-sporangium (2—4 μm in diameter) at the tip, with a chain of 10 or more spores forming a tight spiral coil. The spores have a smooth surface.

(2) State of Growth on Media

In the following description, color standards are bracketed and are in accordance with the Color Harmony Manual of Container Corporation of America.

(a) Sucrose-nitrate agar medium (incubated at 27°C)

Adhering white to light pink [5ba, Shell Pink] aerial mycelia form on an orange [5gc, Peach Tan] to light red [6½ ic, Coral Rose] growth. Slightly purplish soluble pigment forms.

(b) Glucose-asparagine agar medium (incubated at 27°C)

The growth ranges from light yellow [2ea, Lt Wheat—2gc Bamboo] to dull yellow orange [31c, Amber] in color. No adhering aerial mycelia form. No soluble pigment observed.

(c) Glycerin-asparagine agar medium (ISP-medium 5, incubated at 27°C)

The growth is dull orange [3ic, Pastel Orange] in color. No adhering aerial mycelia form. No soluble pigment observed.

(d) Starch-inorganic salt agar medium (ISP-medium 4, incubated at 27°C)

Adhering white to light pink aerial mycelia form on a light reddish orange [5ic, Lt Persimmon] to light red [6ie, Coral Rose] growth. No soluble pigment observed.

(3) Tyrosine agar medium (ISP-medium 7, incubated at 27°C)

The growth ranges from light orange to light yellow brown [3ic, Lt Amber] in color. No adhering aerial mycelia form. No soluble pigment observed.

(f) Nutrient agar medium (incubated at 27°C)

The growth ranges from dull red purple to red purple [6½ ni, Rose Brown] in color. No adhering aerial mycelia form. No soluble pigment observed.

(g) Yeast-malt agar medium (ISP-medium 2, incubated at 27°C)

The growth is dull red [6½ ne, Brick Red]. No adhering aerial mycelia form. No soluble pigment observed.

(h) Oatmeal agar medium (ISP-medium 3, incubated at 27°C)

Adhering white to light pink aerial mycelia form on a light red purple [7ic Colonial Rose] to dull red purple [8le, Rose Wine] growth. The soluble pigment is tinged with red purple. Upon dropwise addition of .5% aqueous NaOH, the growth and soluble pigment turn blue purple, while they turn to the orange color upon addition of 0.5 N aqueous HCl.

(i) Glycerin-nitrate agar medium (incubated at 27°C)

The growth is light reddish orange [4ic, Pastel Orange—5ic, Lt Persimmon]. No adhering aerial mycelia form. No soluble pigment observed.

(j) Starch agar medium (incubated at 27°C)

Thin film of white aerial mycelia forms on a colorless to light pink growth. No soluble pigment observed.

(k) Calcium malate agar medium (incubated at 27°C)

The growth is colorless to yellow brown [3gc, Lt Tan]. No adhering aerial mycelia form. No soluble pigment observed.

(l) Cellulose (synthetic solution containing filter paper chips, incubated at 27°C)

A thin film of white aerial mycelia forms on a light pink [7ec, Rose Mist] growth.

(m) Gelatin stab culture

In incubation on a simple gelatin medium at 20°C, a light orange to dull red [6ic, Coral Rose—7le, Antique Rose] occurs. No adhering aerial mycelia form, and no soluble pigment observed.

In incubation on a glucose-peptone gelatin medium at 27°C, a colorless to light orange growth occurs. No adhering aerial mycelia form and no soluble pigment observed.

(n) Skimmed cow's milk (incubated at 37°C)

The growth is light reddish brown [5ic, Lt Persimmon]. No adhering aerial mycelia form, and no soluble pigment observed.

(3) Physiological Properties

(a) Growth temperature range

Growth test was conducted on starch-inorganic salt agar (ISP-medium 4) at varying temperatures of 20°C, 24°C, 27°C, 30°C, 37°C and 50°C. Except at 50°C, the microorganism grew at each of the temperatures tested, but an optimal temperature would be in the range of 27—37°C.

(b) Liquefaction of gelatin (on 15% simple gelatin at 20°C, and on glucose-peptone gelatin at 27°C)

No liquefaction was observed in 28-day cultivation on simple gelatin or glucose-peptone gelatin.

(c) Hydrolysis of starch (incubated either on starch-inorganic salt agar medium or on starch agar medium at 27°C)

No hydrolysis was observed in 21-day cultivation on the starch-inorganic salt agar medium or on the starch agar medium.

(d) Coagulation and peptonization of skimmed cow's milk (incubated on skimmed cow's milk at 37°C)

Neither coagulation nor peptonization was observed in 28-day incubation.

(e) Formation of melanine pigment (incubated at 27°C on

(i) each or tryptone-yeast broth, ISP-medium 1,

(ii) peptone-yeast-iron agar, ISP-medium 6, and

(iii) tyrosine agar, ISP-medium 7)

Negative on each medium.

(f) Utilization of carbon sources (on Pridham-Gottlieb agar medium, ISP-medium 9, incubated at 27°C)

The microorganism grew utilizing L-arabinose, D-xylose, D-glucose and D-rhamnose, but it would not utilize D-fructose, sucrose, inositol, raffinose or D-mannitol.

(g) Dissolution of calcium malate (on calcium malate agar at 27°C)

No dissolution of calcium malate was observed.

(h) Reduction of nitrate (in aqueous solution of peptone containing 0.1% $KNO_3$, ISP-medium 8, at 27°C)

Positive.

The above observations can be summarized as follows: morphologically, the strain MG463-yF4 has sprial formation on a well extending aerial mycelium and bears a characteristic pseudo-sporangium. The spore has a smooth surface.

It grows on a variety of media, providing light orange to light red growths or a dull red purple growth, with adhering white to light pink aerial mycelia. A red purple soluble pigment is observed on an oatmeal agar medium and the color of the pigment changes with pH. No melanine-like pigment forms, with negative proteolysis and starch hydrolysis.

The strain MG463-yF4 belongs to Type IIIB proposed by Lechevalier et al. (International Journal of Systematic Bacteriology, *20*, 435, 1970) for classification of the predominant components of cell walls, in that the whole cell did contain meso-2,6-diaminopimelic acid and such sugar components as glucose, mannose, ribose and madurose.

In consideration of these facts, the strain MG463-yF4 is assumed to be an Actinomycete of the genus *Actinomadura*. The following two species which are listed in a reference table for the microorganisms of *Actinomadura* (The Biology of the Actinomycetes and Related Organisms, *12*, 30, 1977) seem homologous

to the strain MG463-yF4: they are *Actinomadura roseoviolacea* (Nonomura et al., Hakko Kogaku Zasshi, *49*, 904, 1971) and *Actinomadura carminata* (Gorse et al., Antibiotiki, *8*, 675, 1973).

These two species are believed to be very closely related to each other except for the color of the aerial hypha. According to the reference table mentioned above, *carminata* forms pale lilac to reddish lilac or reddish purple aerial hyphae on oatmeal agar while *roseoviolacea* provides pink red aerial hyphae. It is therefore desirable to conduct experiments for comparing each of these species with the strain MG463-yF4 but the present inventors were unable to obtain a sample of *Actinomadura carminata*.

The following table shows the results of experimental comparison between the strain MG463-yF4 and *Actinomadura roseoviolacea* IMC A-0013 (KCC A-0145). The reported data for *Actinomadura carminata* are reproduced in the right hand column of the table.

TABLE

| | MG463-yF4 | *Actinomadura roseoviolacea*, IMC A-0013 (KCC A-0145) | *Actinomadura carminata* |
|---|---|---|---|
| Morphology of aerial mycelium | Hooked to tight spiral coil, with pseudo-sporangium | Hooked to tight spiral coil, with pseudo-sporangium | Hooked to tight spiral coil, with pseudo-sporangium |
| Spore surface | Smooth | Smooth | Smooth |
| Color of aerial mycelium | white—pale pink | white—pale pink | white—pink |
| Color of growth | dull orange—light red—dull red purple | light orange—light red—dull red purple | light red—red purple—blackish purple |
| Soluble pigment | — to red purple | — to red purple | — to red purple |
| pH dependency of the color of soluble pigment | + NaOH, blue purple | + NaOH, blue purple | |
| | + HCl, orange | + HCl, orange | |
| Formation of melanine-like pigment (ISP-mediums 1, 6 and 7) | — | — | — |
| Hydrolysis of starch | — | — | |
| Coagulation of cow's milk | — | — | |
| Peptonization of cow's milk | — | — (reported as +, weak) | |
| Liquefaction of gelatin | | (reported as +) | |
| simple gelatin | — | — | |
| Utilization of carbon sources | | | |
| D-glucose | + | + | |
| L-arabinose | + | + | |
| D-xylose | (+) | ∓ | |
| D-lactose | ∓ | ∓ | |

TABLE (continued)

| | MG463-yF4 | *Actinomadura roseoviolacea*, IMC A-0013 (KCC A-0145) | *Actinomadura carminata* |
|---|---|---|---|
| Sucrose | ∓ | ∓ | |
| Inositol | ∓ | ∓ | |
| L-rhamnose | + | + | |
| Raffinose | ∓ | ∓ | |
| D-mannitol | ∓ | ∓ | |
| Optimal temperature range | 27 ~ 37°C | 27 ~ 37°C (reported as 30 ~ 40°C) | 28 ~ 30°C |
| Antibiotics produced | carminomycin, rubeomycin, etc. | none reported | carminomycin |

∓: Probably negative

As the above table shows, the strain MG463-yF4 has close resemblance to the *Actinomadura roseoviolacea* strain IMC A-0013 except for the slight difference with respect to the utilization of D-xylose. According to the literature, *Actinomadura roseoviolacea* is weakly positive in terms of gelatin liquefaction and cow's milk peptonization, but in the actual test conducted, negative results were obtained. The literature lacks data for most of the physiological and biochemical properties of *Actinomadura carminata*, a known carminomycin producing microorganism; this species differs from the strain MG463-yF4 with respect to the optimal temperature range, but it is not known whether the properties of the two species differ in other respects. In aspects of morphology and cultivation, no great difference seems to exist between MG463-yF4 and *Actinomadura carminata*. The differences between *Actinomadura carminata* and *Actinomadura roseoviolacea* are also unclear. Therefore, although it is definitely concluded that the strain MG463-yF4 is very closely associated with *Actinomadura roseoviolacea*, the possibility that *Actinomadura carminata* producing a similar antibiotic is also a related species cannot be excluded. It is essential to obtain a sample of *Actinomadura carminata* by some means and to conduct tests for comparing it with the strain MG463-yF4.

With all the observations taken together, the present inventors identified strain MG463-yF4 as being closely related to both *Actinomadura roseoviolacea* and *Actinomadura carminata*.

Production of Barminomycin I and II by Cultivation

The anthracycline compounds, Barminomycin I and II, of the present invention can be produced by aerobically cultivating a barminomycin I- or II-producing microorganism of the genus *Actinomadura* on a suitable medium and recovering the end product from the culture.

The medium may contain any of the nutrient sources that can be utilized by the selected barminomycin I- or II-producing microorganism. Usable carbon sources include glycerin, glucose, sucrose, maltose, dextrin, starch, and fats and oils. Usable nitrogen sources include organics such as soybean meal, cottonseed oil cake, meat extract, peptone, dried yeast, yeast extract and corn steep liquor, and inorganics such as ammonium salts and nitrates, such as, for example, ammonium sulfate, sodium nitrate and ammonium chloride. If necessary, inorganic salts such as sodium chloride, potassium chloride, phosphates and heavy metal salts may be added to the medium. In order to prevent foaming during fermentation, suitable anti-foaming agents such as silicone and soybean oil may be added in suitable amounts as effected in the usual practice.

Aerobic liquid submerged culture is the most appropriate method as is commonly employed in the production of antibiotics. A suitable temperature for cultivation ranges from 20 to 35°C, with the 25—30°C range being preferred. The amount of barminomycin I or II produced by this method, whether it is a shake culture or culture under aeration and agitation, reaches a peak in 3—5 days.

The above procedures will yield a culture in which barminomycin I or II has been accumulated. Part of barminomycin I or II is present in the cells of the cultured microorganism but most of the antibiotic is present in the filtrate of the culture.

Any suitable method may be used to recover barminomycin I or II from the culture. One method depends on the principles of extraction. More specifically, barminomycin I or II in the filtrate of the culture may be extracted with water-immiscible solvents for barminomycin I or II, such as, for example, ethyl acetate, butyl acetate, chloroform and butanol (high extraction efficiency is attained if the filtrate of the culture has a neutral or slightly basic pH). If the antibiotic is present in the cells of the cultured microorganism, the cells are collected by filtration, centrifugation or other suitable techniques and the antibiotic may be recovered by treatment with ethyl acetate, chloroform, methanol, ethanol, butanol, acetone, methyl ethyl ketone, HCl solution or acetic acid solution. The culture may be immediately subjected to the above procedures of extraction without isolating the cells. The cells may be disrupted prior to extraction. The counter current distribution method may be included within the category of extraction techniques.

Another method that can be used to recover barminomycin I or II from the culture depends on the principles of adsorption, wherein an already liquid material containing the desired antibiotic, such as the filtrate of the culture or an extract that has been obtained by the first method, is subjected to column chromatography, liquid chromatography or other analyses using a suitable adsorbent, say, Dia-Ion HP20 (Mitsubishi Chemical Industries, Limited), alumina, silica gel, or "Sephadex LH20" (Pharmacia Fine Chemicals), and the adsorbed antibiotic is eluted with a suitable solvent. The eluate is subsequently concentrated to dryness under vacuum to obtain a crude product of barminomycin I or II as a red powder.

The crude product of barminomycin I or II may be purified by performing the required number of cycles of the extraction and adsorption processes described above, which may be combined as required and optionally followed by recrystallization. Suitable purification techniques that may be used in combination include column chromatography using adsorbents and gel filtration aids such as silica gel, "Sephadex LH20", a weakly acidic ion-exchange resin and "Dia-Ion HP20", liquid chromatography using suitable solvent, counter current distribution, and thin-layer chromatography. A specific purification process may proceed as follows: the crude powder of barminomycin I or II is dissolved in a small amount of chloroform; the solution is loaded onto a silica gel column and fractions are eluted with a suitable solvent; those fractions containing the desired substance are combined and concentrated under vacuum; the concentrate is subjected to thin-layer chromatography and the desired components are recovered to obtain a substantially homogeneous product. This product may be further purified by high-pressure liquid chromatography.

Synthetic Chemical Modification of Barminomycin I and II and Production Thereof

Barminomycin Ir or IIr of the present invention can be produced by reducing the corresponding barminomycin I or II through synthetic chemical means. The reduction reaction may be carried out by any appropriate methods commonly used in organic synthetic chemical fields. For example, barminomycin I or II may be dissolved in methanol and reacted with $NaBH_3CN$. The resulting barminomycin Ir or IIr may be isolated and purified by any suitable method such as chromatography on silica gel that is routinely employed for anthracycline glycosides.

The so obtained barminomycin compounds (I, Ir, II and IIr) may be converted to the corresponding acid addition salts by known methods such as, for example, treatment with an inorganic acid such as hydrochloric acid, sulfuric acid or phosphoric acid, or an organic acid such as acetic acid, propionic acid, maleic acid, oleic acid, palmitic acid, citric acid, succinic acid, tartaric acid, fumaric acid, pantothenic acid, lauric acid, or sulfonic acid.

Uses of Barminomycin

The barminomycin compounds of the present invention have a cancer-control activity and strong anti-microbial activities and are useful as medicines. Some of the biological activities of the barminomycin compounds are described below.

(1) Cytotoxicity (on tissue culture)

The barminomycin compounds of the present invention were capable of inhibiting the growth of cultured P388 leukemia cells in very low concentrations. In addition, these compounds exhibited cytotoxicity levels ranging from 1 to over 100 times the values for adriamycin and other anthracycline compounds of the carminomycin series. For further detail, see the following table.

| | Compound | $IC_{50}$ (µg/ml) |
|---|---|---|
| Compound of the invention | Barminomycin I | ~ 0.00001 |
| | Barminomycin II | ~ 0.00002 |
| | Barminomycin Ir | 0.0015 |
| | Barminomycin IIr | 0.002 |
| Control compound | Carminomycin I | 0.002 |
| | Carminomycin II | 0.010 |
| | Carminomycin III | 0.006 |
| | Adriamycin | 0.013 |

(2) Anti-tumor activity

L1210 leukemia cells ($1 \times 10^5$ per animal) were transplanted intraperitoneally into $CDF_1$ mice. Immediately after the transplantation, 0.25 ml of a selected test compound in solution was administered intraperitoneally in accordance with the schedule shown below. The anti-tumor activities of the test compounds in terms of percentage life prolongation (T/C%) are listed in the same table, with the number of days of survival of control mice to which physiological saline had been administered being taken as 100 (day 0 means the day of L1210 transplantation).

| Dose (µg/mouse/day) | Barminomycin I | Barminomycin II |
|---|---|---|
| 0.25 | Intoxicated to death at 4 days, 120% | Intoxicated to death at 4 days, and 5 days |
| 0.125 | tendency of intoxication, 147% | tendency of intoxication, 173% |
| 0.0625 | 213%, 147% | tendency of intoxication >500%, 173% |
| 0.313 | >500%, 147% | 140% |
| 0.0157 | 187%, 120% | 120% |
| 0.0078 | 160% | >500%, >500% |

Administration schedule: 0—10 days (daily)

| Dose (μg/mouse/day) | Barminomycin Ir | Barminomycin IIr |
|---|---|---|
| 40 | Intoxication, 25% | Intoxication, 37% |
| 20 | Intoxication, 37% | Intoxication, 43% |
| 10 | Tendency of intoxication, 49% | 86% |
| 5 | 141% | 123% |
| 2.5 | 129% | 129% |
| 1.25 | 97% | 123% |

Administration schedule: 0—9 days (daily)

(3) Antimicrobial activity

The antimicrobial activities of barminomycin I and Ir of the present invention, and that of carminomycin III were determined by the agar dilution method. The minimum inhibitory concentration (MIC) data for each antibiotic are listed in the following table.

| | MIC (μg/ml) | | |
|---|---|---|---|
| | Barminomycin I | Barminomycin Ir | Carminomycin III |
| Staphylococcus aureus FAD 209P | 0.2 | 0.78 | 3.12 |
| S. aureus Smith | 0.2 | 0.78 | 3.12 |
| S. aureus MS8710 | 0.2 | 1.56 | 1.56 |
| S. aureus MS9610 | 0.2 | 1.56 | 1.56 |
| Micrococcus Lysodelkticus IFO 3333 | 0.2 | 0.78 | 1.56 |
| Bacillus subtilis PCI 219 | <0.1 | 0.39 | 1.56 |
| E. coli NIHJ | 3.12 | 50 | >50 |
| E. coli K-12 | 0.78 | 6.25 | >50 |
| E. coli BE1121 | <0.1 | 0.2 | 1.56 |
| (E. coli) BE1186 | <0.1 | 0.2 | 1.56 |
| (Klebsiella pneumoniae) PCI 602 | 0.39 | 6.25 | 50 |
| (Serratia marcescens) | 0.78 | 3.12 | 25 |
| (Proteus vulgaris) OX19 | 3.12 | >50 | >50 |
| (Pseudomonas aeruginosa) A3 | 1.56 | 12.5 | >50 |
| (Mycobacterium smegmatis) 607 | 0.78 | 6.25 | 6.25 |

## Experiments

### Example 1

(1) Preparation of seed culture

A medium having the following composition was used.

| | |
|---|---|
| Glucose | 1% |
| Glycerol | 1% |
| Sucrose | 1% |
| Oatmeal | 0.5% |
| Adipron (trademark) | 2% |
| Press-yeast (trademark) | 1% |
| Casamino acids | 0.5% |
| Calcium carbonate | 0.2% |

(pH 7.0 before sterilization)

A portion (100 ml) of this medium was poured into a 500-ml Erlenmeyer flask and sterilized. The medium was then inoculated with a loopful of a slant culture of MG463-yF4. The medium was shake-cultured in a rotary shaker at 30°C for 5 days to prepare a seed culture.

(2) Fermentation

A medium having the following composition was used.

| | |
|---|---|
| Glycerol | 3% |
| Fish meal | 2% |
| Calcium carbonate | 0.2% |

A 30-l jar fermenter was charged with 15 liters of the above specified medium, which was inoculated with 500 ml of the previously prepared seed culture. Cultivation was conducted at 27°C for 48 hours under agitation at 150 rpm and aeration at a rate of 15 l/min.

(3) Recovery of barminomycin I and II

The resulting liquid culture was subjected to filtration and, the filtrate, after pH adjustment to 5.0, was adsorbed on a column (4.0 × 40 cm) of "Dia-Ion HP-20". The column was washed with 6 liters each of water and 50% methanol and eluted with 5 liters of 100% methanol. The eluates were concentrated to obtain 3.0 g of a red crude powder containing both barminomycin I and II.

The powder was dissolved in a small amount of chloroform and the solution was passed through a column packed with 50 g of silica gel (Kieselgel® 60 of Merck). The column was eluted by density gradient of a mixture of chloroform and methanol. The fractions containing barminomycin I and II were concentrated to obtain 40 mg of a red crude powder containing both barminomycin I and II.

Example 2

The red crude powder (40 g) obtained in Example 1 was dissolved in a small amount of chloroform and the solution was spread on 10 thin layers of silica gel (Kieselgel® 60 $F_{254}$ of Merck, 20 × 20 cm), which were developed with a 70:10:1:1 mixture of chloroform, methanol, acetic acid and water. The separating barminomycin I and II fractions were collected and eluted from the silica gel plates with a 2:1 mixture of chloroform and methanol. The so obtained fractions were further purified by high-pressure liquid chromatography on "Nucleosil $_5C_{18}$" using a 40:60 mixture of acetonitrile/0.2% aqueous phosphoric acid as an eluant. The pure red powders of barminomycin I and II were obtained in respective amounts of 3.06 mg and 3.07 mg.

Example 3

A sample (4.5 mg) of the barminomycin I prepared in Example 2 was dissolved in 5 ml of methanol and reacted with 2 mg of $NaBH_3CN$ for 15 minutes. After extraction with 20 ml each of water and chloroform, the chloroform layer was concentrated under vacuum. The concentrate was adsorbed on thin silica gel layers and developed with a 10:1 mixture of chloroform and methanol. The separating barminomycin Ir fractions were collected and eluted from the silica gel plates with a 1:1 mixture of chloroform and methanol. The so obtained fractions were concentrated and loaded on a column (1.0 × 20 cm) of "Sephadex LH20" equilibrated with a 1:1 mixture of chloroform and methanol. The column was eluted with a solution of the same composition. The eluates were evaporated to dryness under vacuum, producing 2.5 mg of barminomycin Ir. The same procedures were repeated to obtain barminomycin IIr from barminomycin II.

4. Brief Description of the Drawings

Fig. 1 is a flow sheet showing the sequence for analyzing the structures of the antibiotic compounds of the present invention;

Fig. 2 is a sketch of an infrared absorption spectrum chart of barminomycin I (in chloroform) of the present invention;

Fig. 3 is a sketch of an infrared absorption spectrum chart of barminomycin II (in chloroform) of the present invention;

Fig. 4 is a sketch of an infrared absorption spectrum chart of barminomycin Ir (as KBr tab.) of the present invention;

Fig. 5 is a sketch of $^1H$-NMR spectrum chart of barminomycin Ir of the present invention;

Fig. 6 is a sketch of an infrared absorption spectrum chart of barminomycin IIr (as KBr tab.) of the present invention;

Fig. 7 is a sketch of $^1H$-NMR spectrum chart of barminomycin IIr of the present invention;

Fig. 8 is a sketch of $^{13}C$-NMR spectrum chart of barminomycin Ir of the present invention.

**Claims for the Contracting States: BE CH DE FR GB IT LI LU NL SE**

1. A compound of the formula I

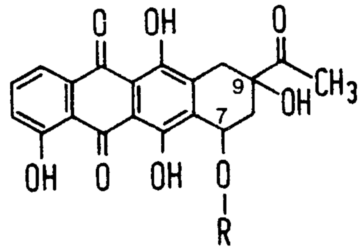

(I)

where R is any one of the following groups (a) to (d)

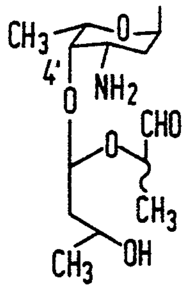

(a)

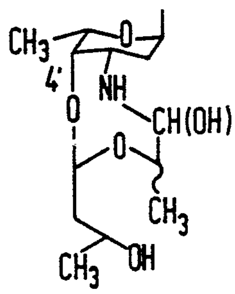

(b)

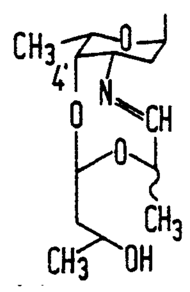

(c)

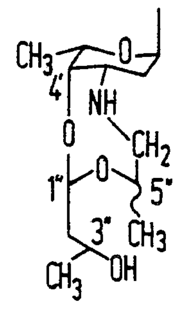

(d)

or acid addition salts thereof.

2. A proces for the preparation of compounds of the formula I as defined in claim 1 wherein R is one of the radicals (a), (b) or (c), which comprises aerobically cultivating a microorganism strain of the genus *Actinomadura* being capable of producing said compound in a nutrient medium containing carbon sources, nitrogen sources and optionally inorganic salts and/or anti-foaming agents, recovering the compounds produced from the culture by extraction with organic solvents and subjecting the extracts to chromatography.

3. The process as defined in claim 2 wherein the strain is cultivated at a temperature of from 20 to 35°C and the cultivation time is 3 to 5 days.

4. The process as defined in claims 2 and 3 wherein the extracts of the cells and/or the culture filtrate are subjected to column chromatography, liquid chromatography or other methods using a suitable absorbent.

5. The process as defined in claims 2 to 4 wherein the strain MG 463-yF4 (FERM P No. 7352) (FERM—BP—1044) of the genus *Actinomadura* is used.

6. A process for the preparation of compounds of the formula I as defined in claim 1, wherein R is the radical

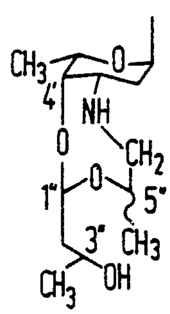

(d)

which comprises reducing a compound of the formula I wherein R is the radical

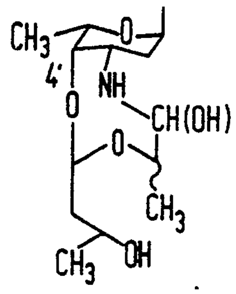 or 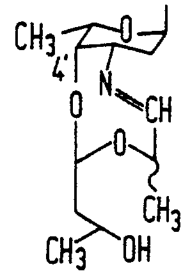

(b) (c)

with $NaBH_3CN$.

7. A pharmaceutical composition which comprises as active ingredient a compound as defined in claim 1 in association with a pharmaceutically acceptable carrier.

8. Use of a compound as defined in claim 1 for the preparation of a medicament having anti-tumor and anti-microbial activities.

**Claims for the Contracting State: AT**

1. A process for the preparation of compounds of the formula I

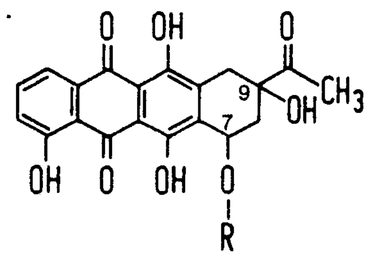

(I)

where R is any one of the following groups (a) to (c)

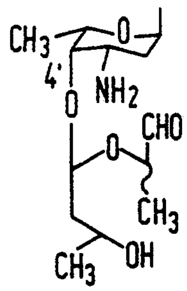 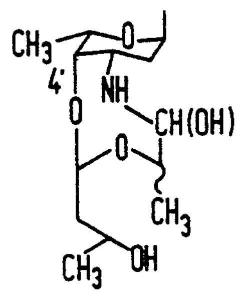 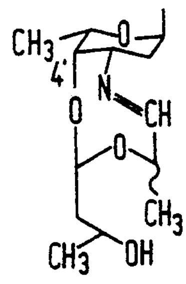

(a) (b) (c)

which comprises aerobically cultivating a microorganism strain of the genus *Actinomadura* being capable of producing said compound in a nutrient medium containing carbon sources, nitrogen sources and optionally inorganic salts thereof and/or anti-foaming agents, recovering the compounds produced from the culture by extraction with organic solvents and subjecting the extracts to chromatography.

2. A process as defined in claim 1 wherein the strain is cultivated at a temperature of from 20 to 35°C and the cultivation time is 3 to 5 days.

3. The process as defined in claims 1 and 2 wherein the extracts of the cells and/or the culture filtrate are subjected to column chromatography, liquid chromatography or other methods using a suitable absorbent.

4. The process as defined in claims 1 to 3 wherein the strain MG 463-yF4 (FERM P—No. 7352) (FERM—BP—1044) is used.

5. A process for the preparation of compounds of the formula I as defined in claim 1, wherein R is the radical

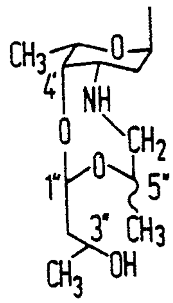

(d)

which comprises reducing a compound of the formula I wherein R is the radical

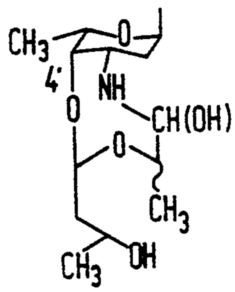

or

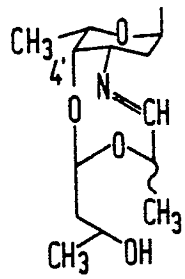

(b) (c)

with $NaBH_3CN$.

**Patentansprüche für die Vertragsstaaten: BE CH DE FR GB IT LI LU NL SE**

1. Eine Verbindung der Formel I

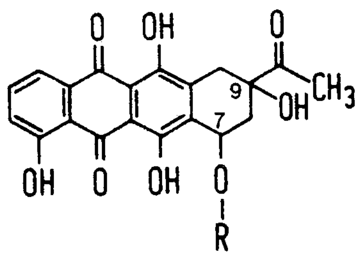

(I)

wobei R für irgendeine der folgenden Gruppen (a) bis (d) steht

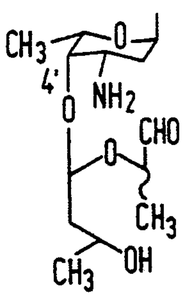

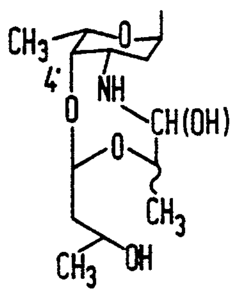

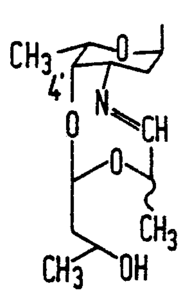

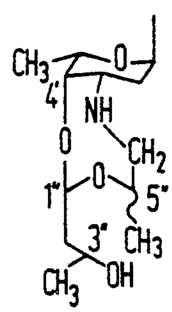

(a) (b) (c) (d)

oder deren Säureadditionssalze.

2. Ein Verfahren zur Herstellung von Verbindungen der Formel I wie in Anspruch 1 definiert, worin R eines der Radikale (a), (b) oder (c) bedeutet, gekennzeichnet durch die aerobe Kultur eines Mikroorganismenstammes der Gattung *Actinomadura,* der fähig ist, die genannte Verbingung in einem Nährboden der Kohlenstoffquellen, Stickstoffquellen und wahlweise anorganische Salze und/oder

Antischaummittel enthält, zu bilden, die Rückgewinnung der gebildeten Verbindungen aus der Kultur durch Extraktion mit organischen Lösungsmitteln und Durchführung der Chromatographie an den Extrakten.

3. Das wie in Ausspruch 2 definierte Verfahren, worin der Stamm bie Temperaturen von 20 bis 35°C gezüchtet wird und die Züchtungsdauer der Kultur 3 bis 5 Tage beträgt.

4. Das wie in den Ansprüchen 2 und 3 definierte Verfahren, worin die Extrakte der Zellen und/oder das Kulturfiltrat der Säulenchromatographie, der Flüssigchromatographie oder anderen Methoden, bei denen ein geeignetes Adsorptionsmittel verwendet wird, unterzogen werden.

5. Das wie in den Ansprüchen 2 bis 4 definierte Verfahren, worin der Stamm MG463-yF4 (FERM P No. 7352) (FERM—BP—1044) der Gattung *Actinomadura* verwendet wird.

6. Ein Verfahren zur Herstellung von Verbindungen der wie in Anspruch 1 definierten Formel I, worin R das Radikal

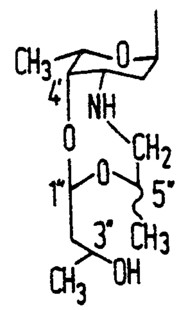

(d)

bedeutet, gekennzeichnet durch die Reduktion einer Verbindung der Formel I, worin R das Radikal

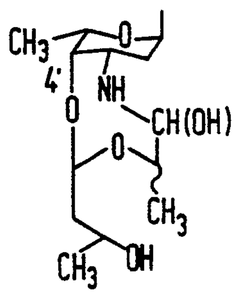

oder

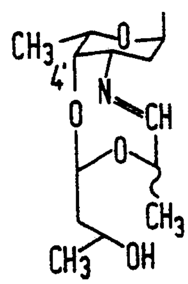

(b) (c)

bedeutet, mit $NaBH_3CN$.

7. Eine Pharmazeutische Zusammensetzung, dadurch gekennzeichnet, daß sie als Wirkstoff eine wie in Anspruch 1 definierte Verbindung enthält, zusammen mit einem pharmazeutisch unbedenklichen Träger.

8. Verwendung einer wie in Anspruch 1 definierten Verbindung zur Herstellung eines Medikaments mit antitumorieller und antimikrobieller Wirkung.

**Patentansprüche für den Vertragsstaat: AT**

1. Ein Verfahren zur Herstellung von Verbindungen der Formel I

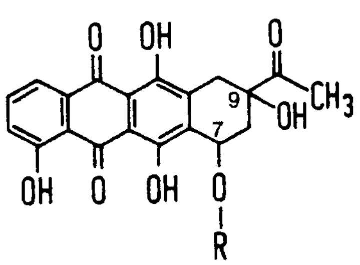

(I)

wobei R für irgendeine der folgenden Gruppen (a) bis (c) steht

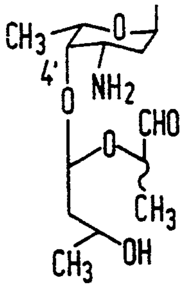

(a)

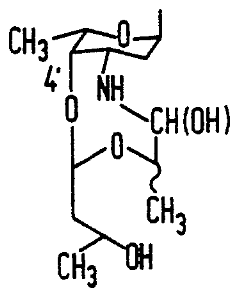

(b)

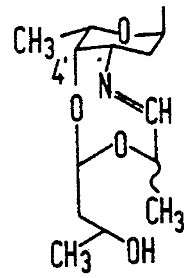

(c)

gekennzeichnet durch die aerobe Kultur eines Mikroorganismenstammes der Gattung *Actinomadura*, der fähig ist, die genannte Verbindung in einem Nährboden, der Kohlenstoffquellen, Stickstoffquellen und wahlweise anorganische Salze und/oder Antischaummittel enthält, zu bilden, die Rückgewinnung der gebildeten Verbindungen aus der Kulter durch Extraktion mit organischen Lösungsmitteln und Durchführung der Chromatographie an den Extrakten.

2. Das wie in Anspruch 1 definierte Verfahren, worin der Stamm bei Temperaturen von 20 bis 35 °C gazüchtet wird und die Züchtungsdauer der Kultur 3 bis 5 Tage beträgt.

3. Das wie in den Ansprüchen 1 und 2 definierte Verfahren, worin die Extrakte der Zellen und/oder das Kulturfiltrat der Säulenchromatographie, der Flüssigchromatographie oder anderen Methoden, bei denen ein geeignetes Adsorptionsmittel verwendet wird, unterzogen werden.

4. Das wie in den Ansprüchen 1 bis 3 definierte Verfahren, worin der Stamm MG463-yF4 (FERM P No. 7352) (FERM—BP—1044) der Gattung *Actinomadura* verwendet wird.

5. Ein Verfahren zur Herstellung von Verbindungen der wie in Anspruch 1 definierten Formel I, worin R das Radikal

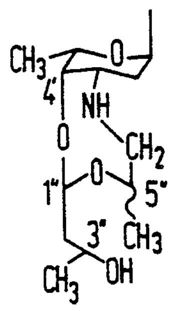

(d)

bedeutet, gekennzeichnet durch die Reduktion einer Verbindung der Formel I, worin R das Radikal

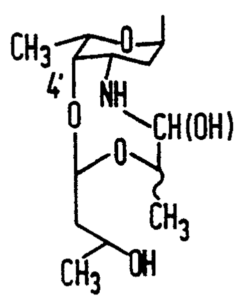

oder

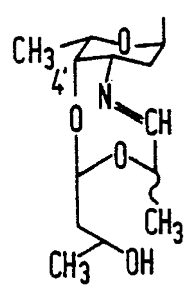

(b) (c)

bedeutet, mit $NaBH_3CN$.

**Revendications pour les Etats contractants: BE CH DE FR GB IT LI LU NL SE**

1. Composé de formule I

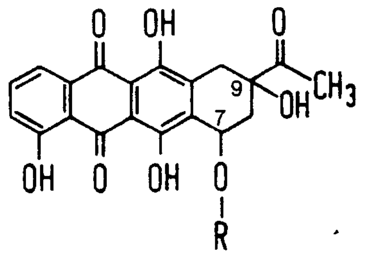

(I)

dans laquelle R est l'un quelconque des groupes (a) à (d) suivants

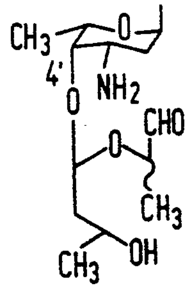

(a)

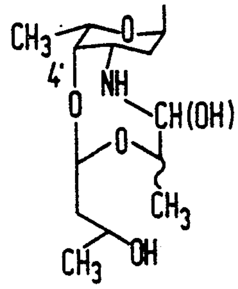

(b)

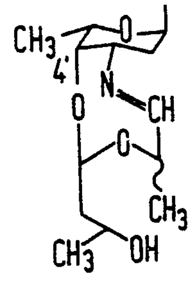

(c)

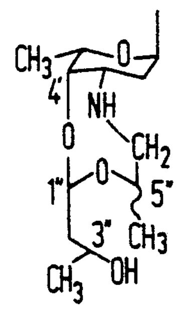

(d)

ou ses sels d'addition avec des acides.

2. Procédé pour la préparation des composés de formule I comme défini dans la revendication 1, dans laquelle R est un des radicaux (a), (b) ou (c), qui comprend la culture aérobie d'une souche de micro-organismes du genre *Actinomadura* capable de produire ledit composé dans un milieu nutritif contenant des sources de carbone, des sources d'azote et, facultativement, des sels minéraux et/ou des agents antimousse, la récupération des composés, produits par la culture, par extraction avec des solvants organiques et la chromatographie des extraits.

3. Procédé selon la revendication 2, dans lequel la souche est cultivée à une température de 20 à 35°C et la durée de culture est de 3 à 5 jours.

4. Procédé selon les revendications 2 et 3, dans lequel les extraits des cellules et/ou le filtrat de culture sont soumis à une chromatographie sur colonne, une chromatographie liquide ou à d'autres procédés utilisant un adsorbant approprié.

5. Procédé selon les revendications 2 à 4, dans lequel on utilise la souche MG 463-yF4 (FERM P No. 7352) (FERM—BP-1044) du genre *Actinomadura*.

6. Procédé pour la préparation de composés de formule I selon la revendication 1, dans laquelle R est le radical

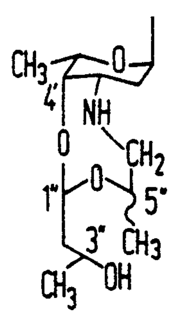

(d)

qui comprend la réduction d'un composé de formule I dans laquelle R est le radical

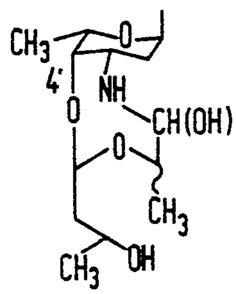 ou 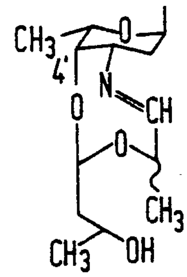

(b) (c)

avec $NaBH_3CN$.

7. Composition pharmaceutique qui comprend comme ingrédient actif un composé comme défini dans la revendication 1 en association avec un véhicule pharmaceutiquement acceptable.

8. Utilisation d'un composé selon la revendication 1 pour la préparation d'un médicament ayant des activités antitumorale et antimicrobienne.

**Revendications pour l'Etat contractant: AT**

1. Procédé pour la préparation de composés de formule I

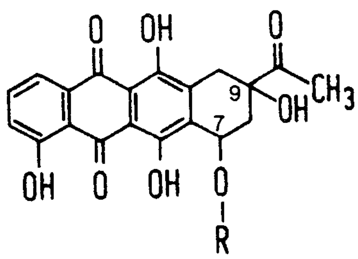

(I)

dans laquelle R est l'un quelconque des groupes (a) à (c) suivants

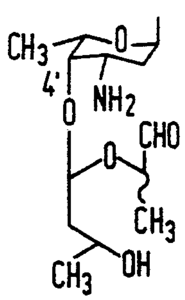

(a)

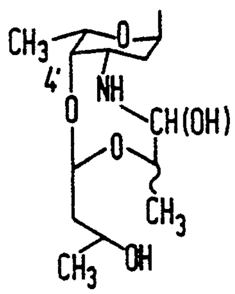

(b)

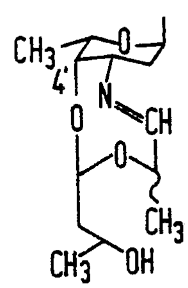

(c)

qui comprend la culture aérobie d'une souche de micro-organismes du genre *Actinomadura* capable de produire ledit composé dans un milieu nutritif contenant des sources de carbone, des sources d'azote et, facultativement, des sels minéraux et/ou des agents antimousse, la récupération des composés, produits par la culture, par extraction avec des solvants organiques et la chromatograpie des extraits.

2. Procédé selon la revendication 1, dans lequel la souche est cultivée à une température de 20 à 35°C et la durée de culture est de 3 à 5 jours.

3. Procédé selon les revendications 1 et 2, dans lequel les extraits des cellules et/ou le filtrat de culture sont soumis à une chromatographie sur colonne, une chromatographie liquide ou à d'autres procédés utilisant un adsorbant approprié.

4. Procédé selon les revendications 1 à 3, dans lequel on utilise la souche MG 463-yF4 (FERM P No. 7352) (FERM—BP-1044).

5. Procédé pour la préparation de composés de formule I selon la revendication 1, dans laquelle R est le radical

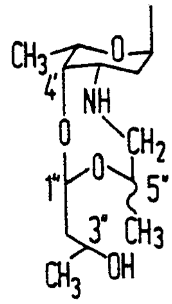

(d)

qui comprend la réduction d'un composé de formule I dans laquelle R est le radical

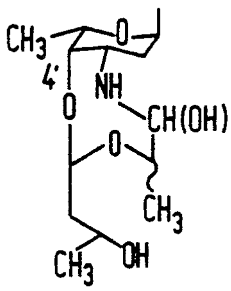

ou

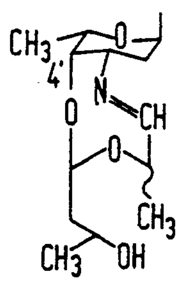

(b) (c)

avec $NaBH_3CN$.

FIG. 1

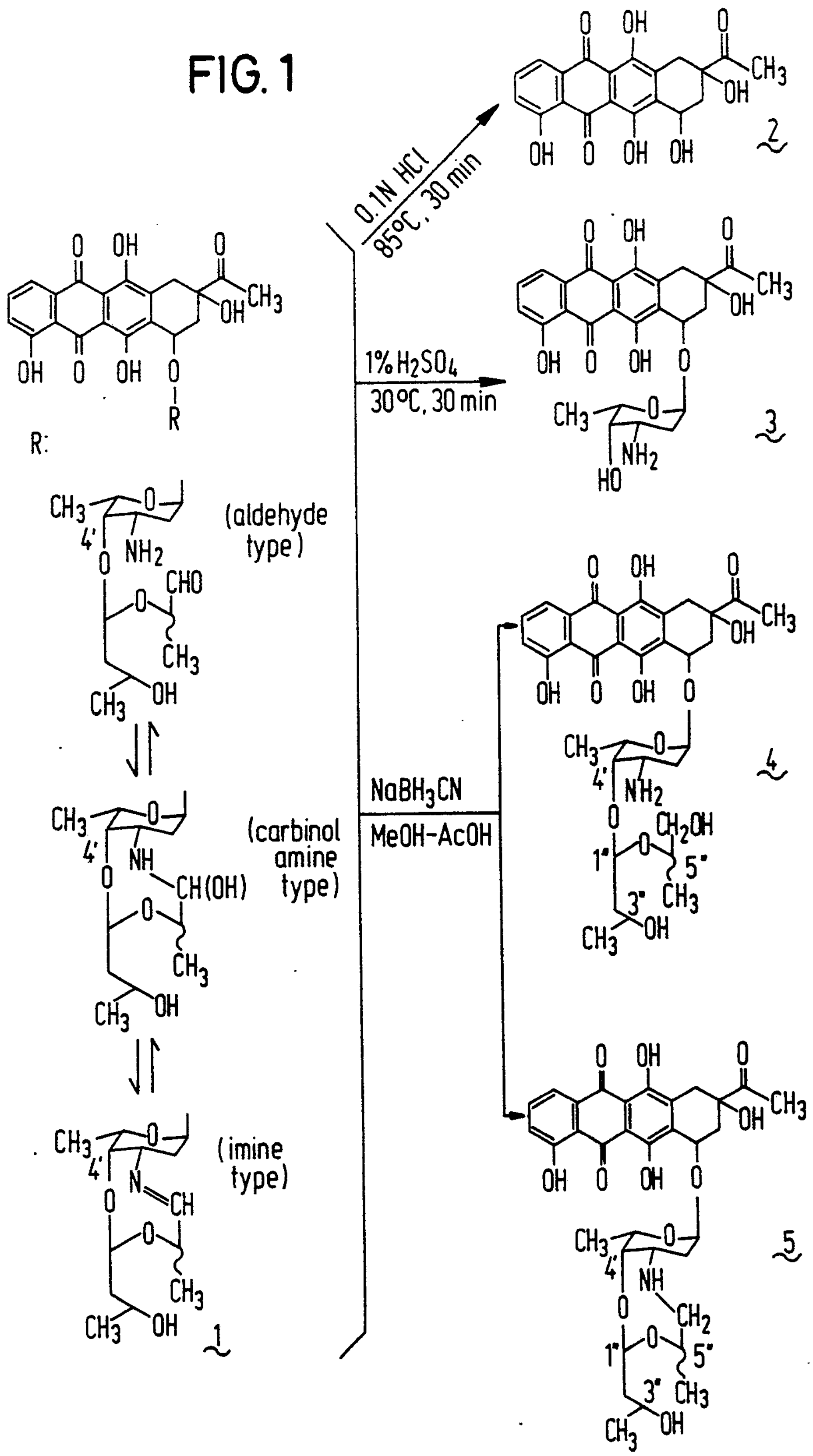
0.1N HCl
85°C, 30 min
1% H2SO4
30°C, 30 min
NaBH3CN
MeOH-AcOH
(aldehyde type)
(carbinol amine type)
(imine type)

1 2 3 4 5

FIG. 2

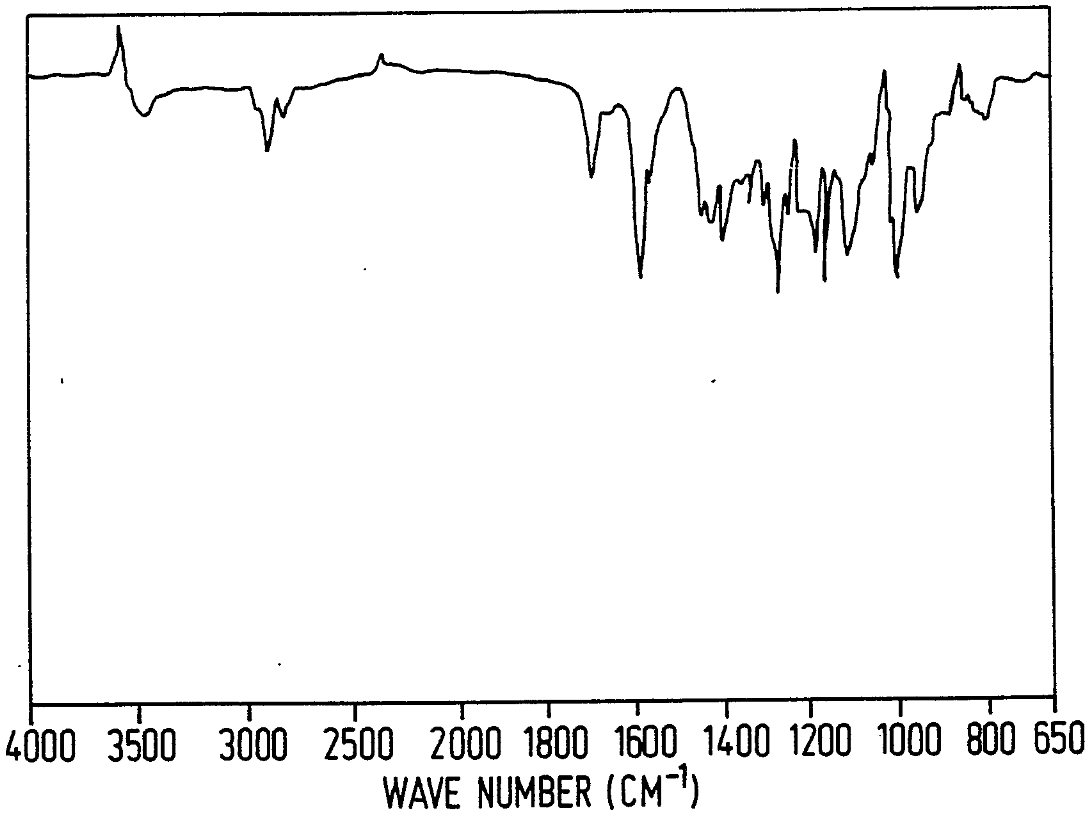
4000
3500
3000
2500
2000
1800
1600
1400
1200
1000
800
650
WAVE NUMBER ($CM^{-1}$)

FIG. 3

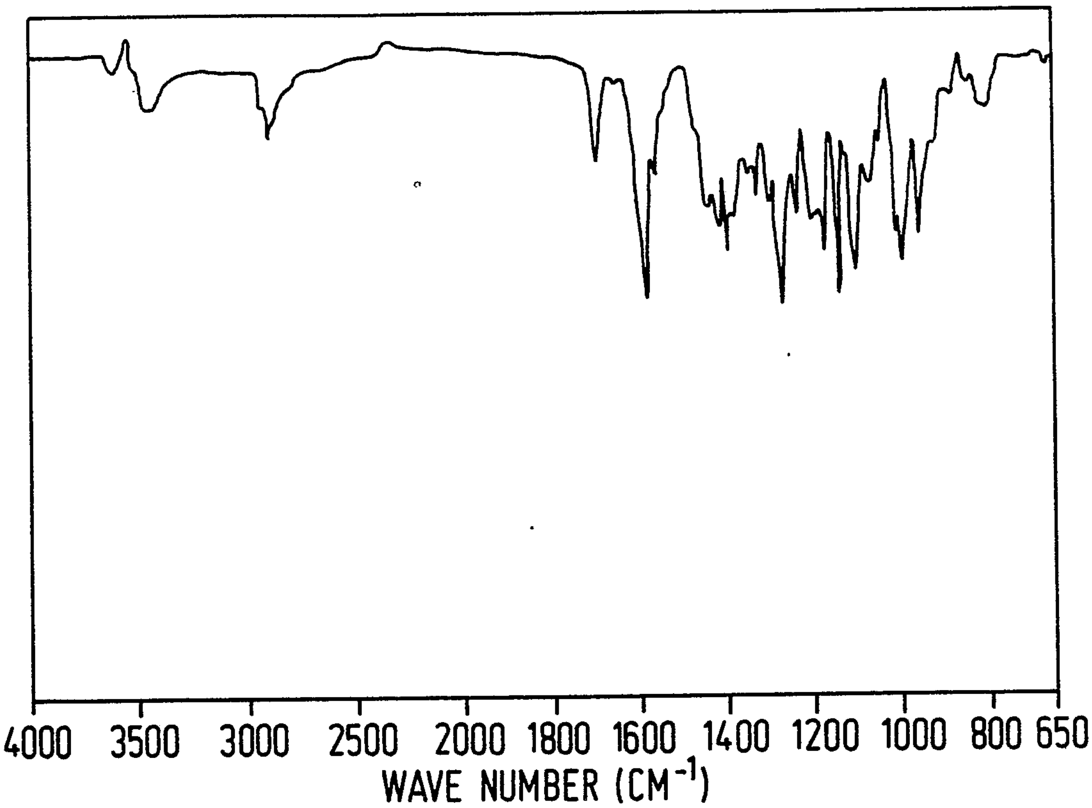
4000 3500 3000 2500 2000 1800 1600 1400 1200 1000 800 650
WAVE NUMBER (CM$^{-1}$)

FIG. 4

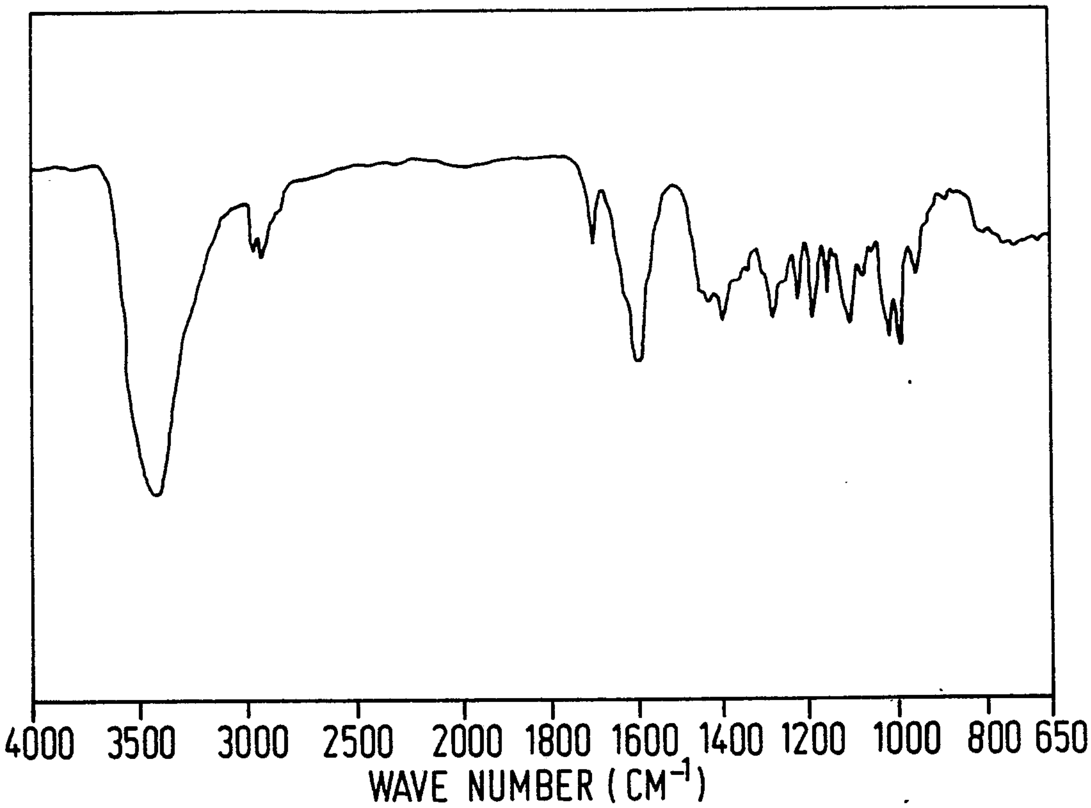
4000 3500 3000 2500 2000 1800 1600 1400 1200 1000 800 650
WAVE NUMBER (CM⁻¹)

FIG.5

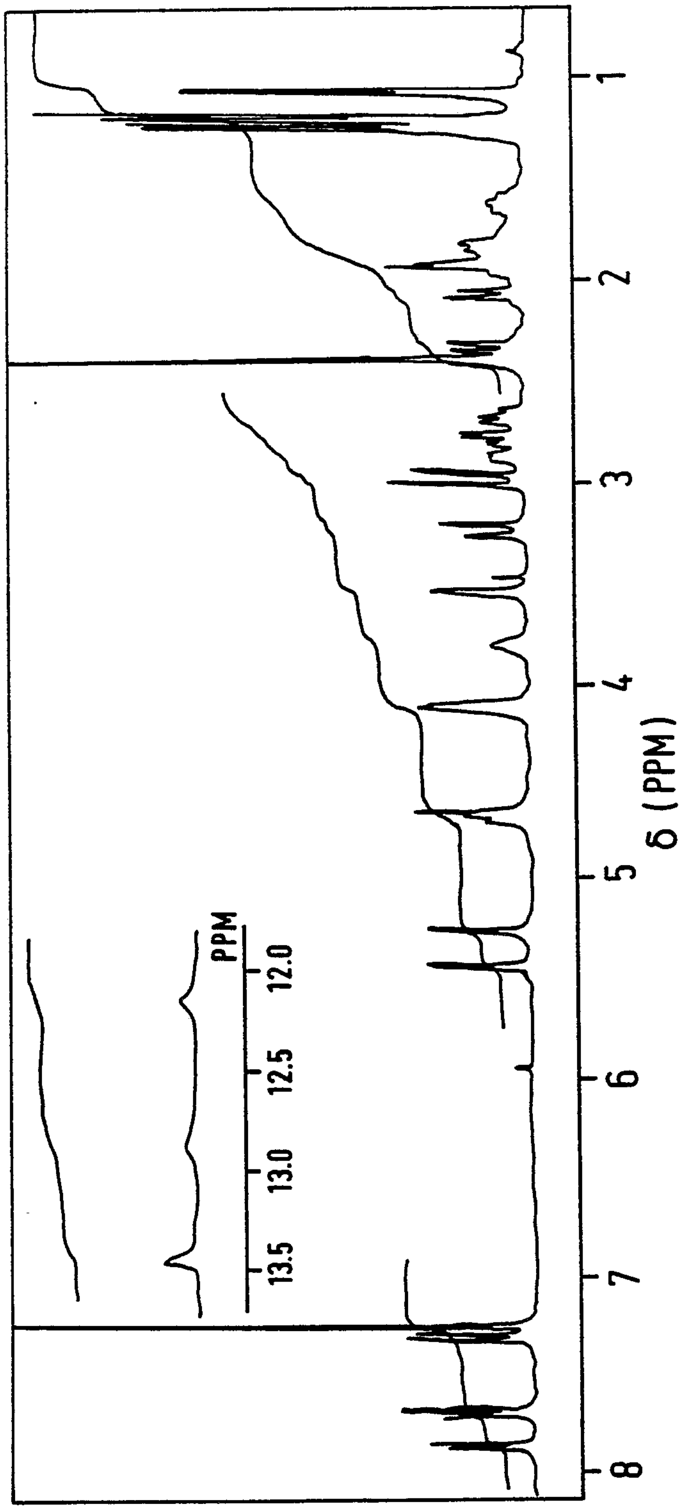

FIG.6

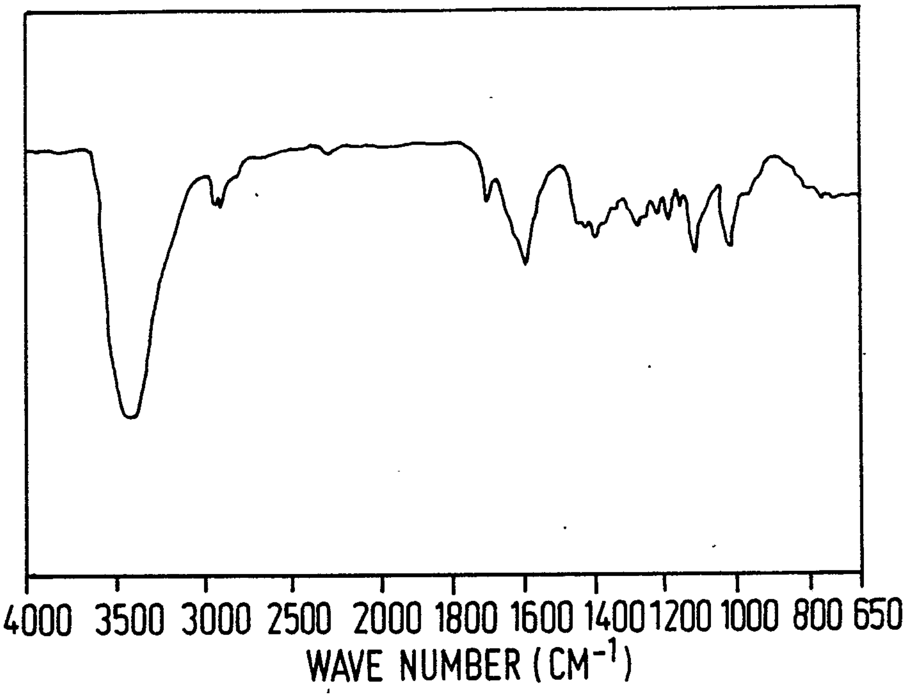
4000
3500
3000
2500
2000
1800
1600
1400
1200
1000
800
650
WAVE NUMBER (CM-1)

FIG.7

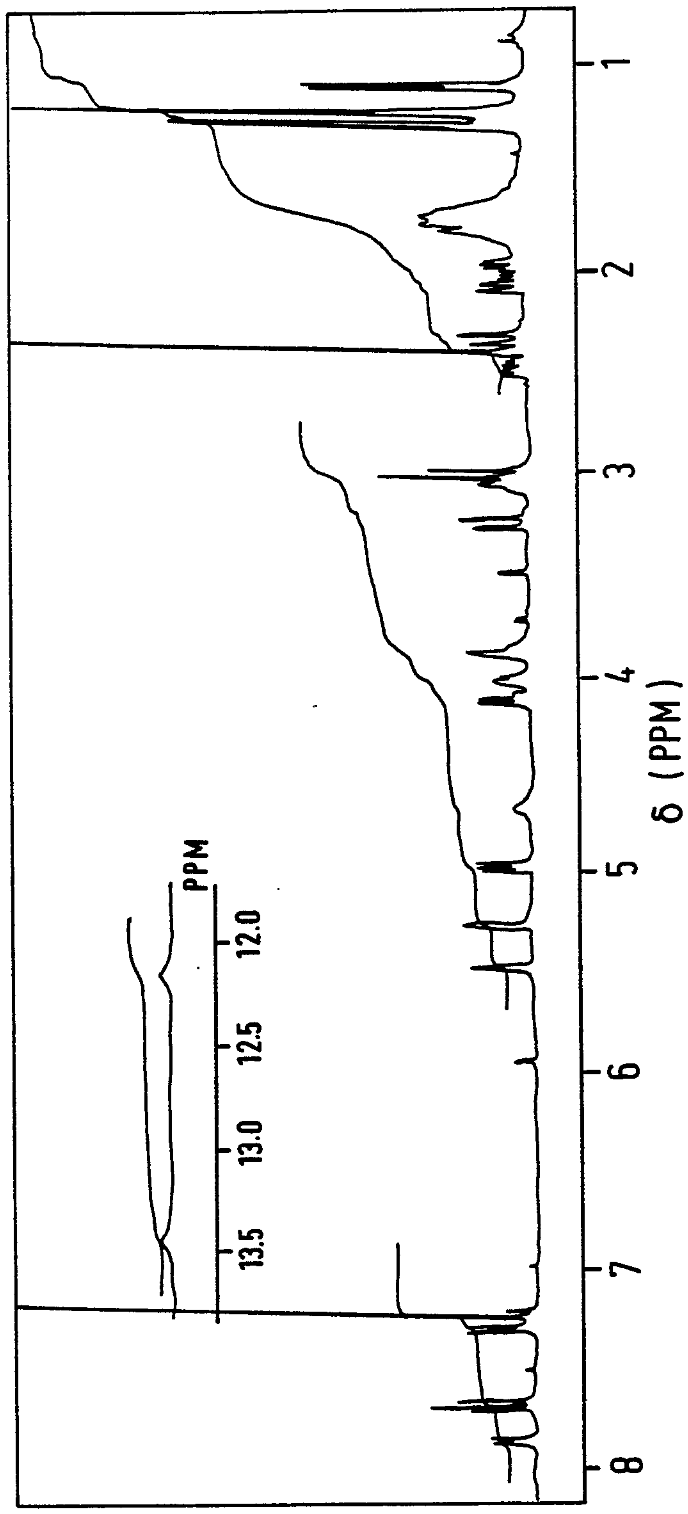

FIG. 8

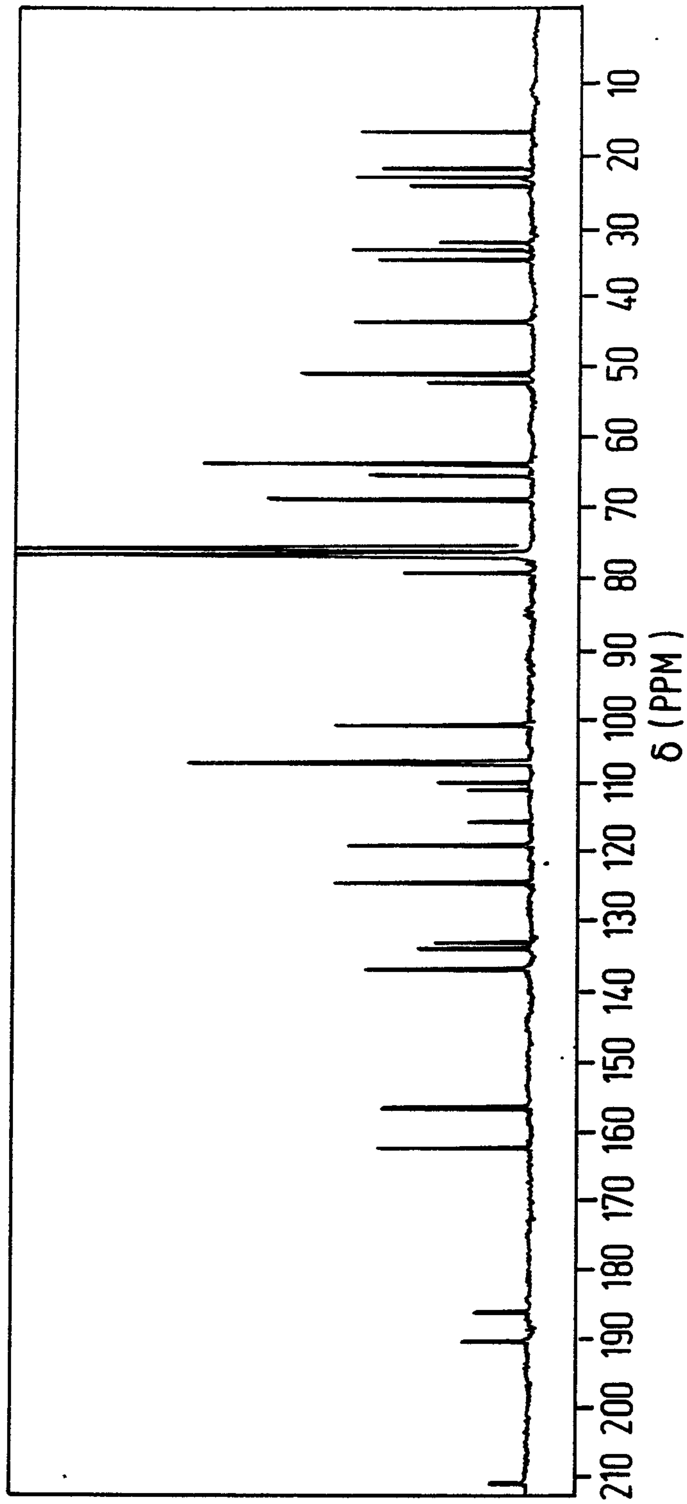
10
20
30
40
50
60
70
80
90
100
110
120
130
140
150
160
170
180
190
200
210
δ (PPM)